# EUROPEAN PATENT APPLICATION

(11) **EP 3 352 012 A1**
(43) Date of publication of application: **25.07.2018**
(21) Application number: 17187787.1
(22) Date of filing: 24.08.2017
(51) Int. Cl.: G03B 42/00, A61B 6/00, G03B 42/04

(54) **SENSOR HOLDER FOR INTRAORAL X-RAY SENSOR**

(30) Priority: 24.01.2017 KR 20170010813
(71) Applicant: Rayence Co., Ltd., Hwaseong-si, Gyeonggi-do 445-170 (KR); Vatech Ewoo Holdings Co., Ltd., Hwaseong-si, Gyeonggi-do 445-170 (KR)
(72) Inventor: Heo, Sungkyn, 445-170 Gyeonggi-do (KR); Park, Seungun, 445-170 Gyeonggi-do (KR)
(74) Representative: Markfort, Iris-Anne Lucie

(57) **Abstract**

Disclosed is a sensor holder for an intraoral X-ray sensor including a front surface receiving incident x-rays, a rear surface, and a fastener, including a coupler including a grip fitted over a side surface of the fastener to enclose the same, and a support connected to the grip and facing in a forward direction of the intraoral X-ray sensor while covering the rear surface and a side surface of the intraoral X-ray sensor, a connector connected to the support and extending in the forward direction of the intraoral X-ray sensor, and a handle connected to the connector, wherein the coupler is made of a synthetic resin material with a first hardness (L1), and at least one of the connector and the handle is made of a synthetic resin material with a second hardness (L2) greater than the first hardness.

## Description

### TECHNICAL FIELD

The present invention relates generally to a sensor holder for an intraoral X-ray sensor. More particularly, the present invention relates to a sensor holder that is capable of being physically coupled to and decoupled from an intraoral X-ray sensor that is inserted into a mouth, in order to place the intraoral X-ray sensor at a desired location when intraoral radiography is performed.

### BACKGROUND ART

Generally, in order to perform intraoral radiography on a subject in a dental clinic, an intraoral X-ray sensor is engaged with an instrument called a sensor holder and then is inserted into a subject's mouth. After that, X-rays are then projected onto the intraoral X-ray sensor from an X-ray irradiation device provided outside the subject's mouth, whereby an intraoral structure between the X-ray irradiation device and the intraoral X-ray sensor is radiographed.

Herein, the intraoral sensor is an image sensor that generates electric signals that projection data of the subject is reflected on by sensing X-rays having passed through a subject; and the sensor holder is an assistant tool configured to place the intraoral X-ray sensor at a desired location inside the subject's mouth, and to arrange the intraoral X-ray sensor and the X-ray irradiation device to be arrayed to face each other along a straight line when it is necessary.

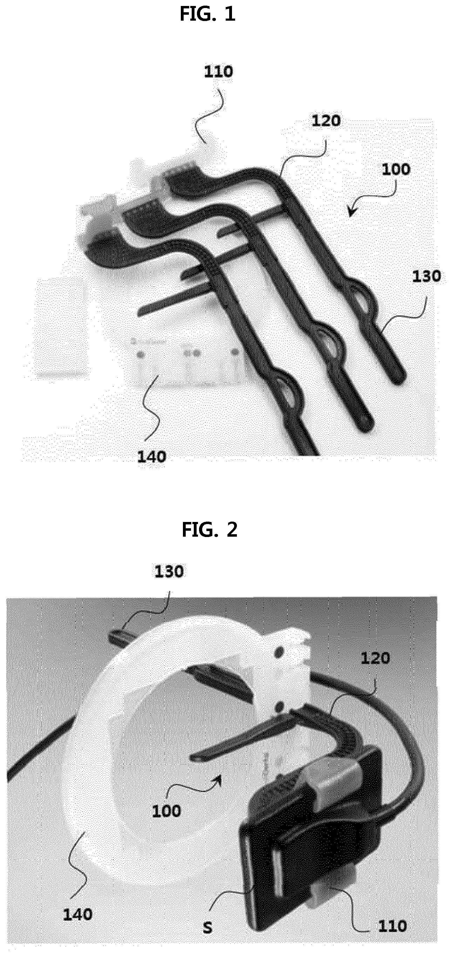

FIG. 1 is a view showing a combined state of a conventional sensor holder 100 and an intraoral X-ray sensor S.

As shown in the drawing, the conventional sensor holder 100, which is in a predetermined bar shape, includes: a coupler 110 allowing an intraoral X-ray sensor S to be coupled thereto; a connector 120 extending from the coupler 110; and a handle 130 extending from the connector 120, wherein the connector 120 is integrally or detachably connected with a ring 140 that faces the intraoral X-ray sensor S in parallel.

Accordingly, a user performs intraoral radiography in the following manner: the intraoral X-ray sensor S is engaged with the coupler 110 of the sensor holder 100; then the intraoral X-ray sensor S is inserted into the subject's mouth to be placed at a desired location while the user holds the handle 130; X-rays are then emitted onto the intraoral X-ray sensor S through the ring 140, which is outside the mouth. For reference, FIG. 2 is a photograph showing examples of the conventional holder, wherein a shape of the sensor holder 100 may vary according to a target structure inside a subject's mouth, that is, a canine, an anterior tooth, a molar tooth, and the like, and generally, different shapes of holders are formed as a set.

Meanwhile, the coupler 110 of the conventional sensor holder 100 is configured such that a part thereof grips a part of a rear surface of the intraoral X-ray sensor S by extending over an edge thereof while covering a front surface, as an X-ray incident surface, of the intraoral X-ray sensor S; and the user couples the intraoral X-ray sensor S to the back of the coupler 110, such that the front surface of the intraoral X-ray sensor S faces the handle 130.

For this reason, the coupler 110 of the conventional sensor holder 100 is placed in an X-ray path when intraoral radiography is performed, thereby interfering with the front surface, as an X-ray receiving surface, of the intraoral X-ray sensor S, and as a result, quality of an X-ray image may be degraded since the coupler 110 of the sensor holder 100 may be reflected in the X-ray image.

The conventional sensor holder 100 is further problematic in that since the coupler 110 thereof is configured to extend over the edge of the intraoral X-ray sensor S to grip a part of the rear surface from the front surface of the intraoral X-ray sensor S, a size of the part of the coupler inserted into the subject's mouth is increased, thereby increasing inconvenience to the subject. The conventional sensor holder 100 is further problematic in that in the process of coupling or decoupling the coupler 110 to or from the intraoral X-ray sensor S, undesirable shock may be exerted onto the X-ray receiving surface, as a main part of the intraoral X-ray sensor S, etc., thereby causing damage.

The conventional sensor holder 100 is further problematic in that since the entire structure except the ring 140 is made of a hard metal or a synthetic resin material, it may cause pain to the subject by strongly pressing the subject's gums.

The foregoing is intended merely to aid in the understanding of the background of the present invention, and is not intended to mean that the present invention falls within the purview of the related art that is already known to those skilled in the art.

### SUMMARY OF INVENTION

Accordingly, the present invention has been made keeping in mind the above problems occurring in the prior art, and an object of the present invention is to provide a sensor holder that is capable of securely fastening an intraoral X-ray sensor without interfering with an X-ray receiving surface, and is capable of minimizing inconvenience to a subject when intraoral radiography is performed by minimizing sizes of elements including the intraoral X-ray sensor that are inserted into the subject's mouth.

Another object of the present invention is to provide a sensor holder that is capable of reducing shock that may be exerted onto an intraoral X-ray sensor when the intraoral X-ray sensor is coupled to or decoupled from the sensor holder, while facilitating disassembly and assembly thereof, and is capable of minimizing pain to the subject when a user strongly presses the subject's gums by poorly handling the sensor holder.

In order to achieve the above object, according to one aspect of the present invention, there is provided a sensor holder for an intraoral X-ray sensor including a front surface receiving incident x-rays, a rear surface opposite to the front surface, and a fastener provided by protruding rearward on the rear surface, the sensor holder including a coupler including a grip configured to be fitted over a side surface of the fastener to enclose at least a part of the side surface, and a support configured to be connected to the grip and to face in a forward direction of the intraoral X-ray sensor while covering at least parts of the rear surface and a side surface of the intraoral X-ray sensor, a connector configured to be connected to the support, and to extend in the forward direction of the intraoral X-ray sensor, and a handle configured to be connected to the connector, wherein the coupler is made of a synthetic resin material with a first hardness L1, and at least one of the connector and the handle is made of a synthetic resin material with a second hardness L2 greater than the first hardness.

Here, particularly, the first hardness may be Shore hardness between D30 and D70, and the second hardness may be Shore hardness of D80 or more.

Further, the connector connected to the support may be configured such that at least a part thereof has a step that is higher than the connector to cover a part of an edge of a front surface of the intraoral X-ray sensor, wherein the step is inclined such that a height thereof is gradually decreased from the connector to the support.

Further, the intraoral X-ray sensor may further include a cable withdrawn from a side surface of the fastener, and the grip may be configured such that a part thereof is open in a direction of the side surface of the fastener.

Further, the fastener of the intraoral X-ray sensor may be provided with a grip groove on at least a part of a side surface thereof, and the grip may be configured to enclose the fastener along the grip groove.

The sensor holder according to the present invention is advantageous in that since the sensor holder is coupled to a fastener provided in a rear surface of an intraoral X-ray sensor, it is possible to securely fasten the intraoral X-ray sensor without interfering with an X-ray incident surface, and it is possible to minimize inconvenience to a subject when intraoral radiography is performed, by minimizing sizes of elements including the intraoral X-ray sensor that are inserted into the subject's mouth.

The sensor holder according to the present invention is further advantageous in that since an inclined surface between a coupler and a connector is provided, it is possible to reduce shock that may be exerted onto an intraoral X-ray sensor when the intraoral X-ray sensor is coupled to or decoupled from the sensor holder, while facilitating disassembly and assembly thereof, and since the coupler is made of a synthetic resin with hardness lower than the other elements, it is possible to minimize pain to a subject even when the subject's gums are strongly pressed.

### BRIEF DESCRIPTION OF DRAWINGS

The above and other objects, features and other advantages of the present invention will be more clearly understood from the following detailed description when taken in conjunction with the accompanying drawings, in which:
FIG. 1 is a view showing a combined state of a conventional sensor holder and an intraoral X-ray sensor;
FIG. 2 is a view showing a set of the conventional sensor holder;
FIG. 3 is a sectional view showing the intraoral X-ray sensor coupled to a sensor holder according to the present invention;
FIGS. 4 and 5 are views respectively showing the sensor holder according to the present invention;
FIG. 6 is a view showing a combined state of the sensor holder according to the present invention, and the intraoral X-ray sensor; and
FIG. 7 is a view showing a set of the sensor holder according to the present invention.

### DESCRIPTION OF EMBODIMENTS

The terminologies or words used in the description and the claims of the present invention should not be interpreted as being limited merely to their common and dictionary meanings. On the contrary, they should be interpreted based on the meanings and concepts of the invention in keeping with the scope of the invention based on the principle that the inventor(s) can appropriately define the terms in order to describe the invention in the best way.

Hereinbelow, exemplary embodiments of the present invention will be described in detail with reference to the accompanying drawings. Throughout the drawings, the same reference numerals will refer to the same or like parts.

Before description of the present invention, a sensor holder according to the present invention is configured to be connected to an X-ray receiving surface of an intraoral X-ray sensor, that is, to a rear surface opposite to a front surface. To achieve this, the intraoral X-ray sensor includes a front surface on which x-rays are incident, a rear surface opposite to the front surface, and particularly, a fastener provided by protruding rearward on the rear surface.

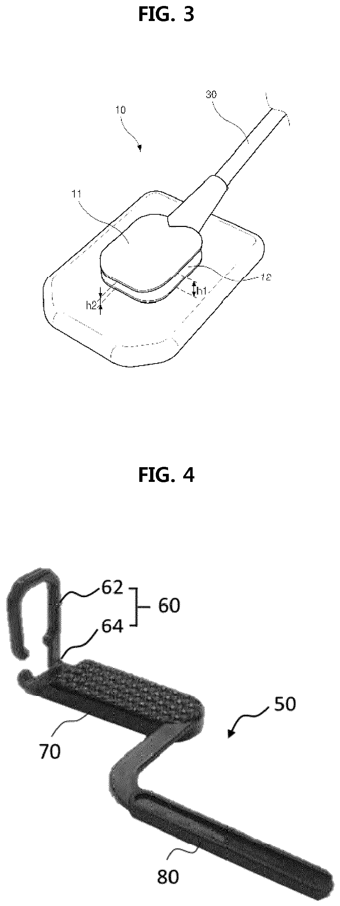

FIG. 3 is a sectional view showing the intraoral X-ray sensor 10 coupled to the sensor holder according to the present invention.

As shown in the drawing, the intraoral X-ray sensor 10 is an image sensor, which is inserted into a subject's mouth when intraoral radiography is performed, and generates electric signals that projection data of the subject is reflected on by receiving X-rays having passed through a subject. To achieve this, the intraoral X-ray sensor 10 includes: an image sensor panel configured to generate electric signals by receiving X-rays; a circuit configured to supply external power to the image sensor panel, and to process the electric signals generated from the image sensor panel; a cable 30 provided for internal and external communication and power supply of the intraoral X-ray sensor 10; and a housing configured to cover the image sensor panel, the circuit, and the like, except a part of the cable 30.

Particularly, the intraoral X-ray sensor 10 further includes a protruding grip post, namely, a fastener 11, which is provided by protruding rearward on the rear surface that is opposite to the front surface on which X-rays are incident, such that the cable 30 is withdrawn through a side surface of the fastener 11 to the outside.

The fastener 11 may be formed into a quadrangular shape from a plan view, but is not limited thereto. The fastener may be in a polygonal shape, or in an annular shape. Hereinafter, for the convenience of illustration, a case where the fastener is in a quadrangular shape is used as an example, and as shown in the accompanying drawings, edges thereof may be rounded.

The fastener 11 may be provided with a grip groove 12 on at least a part of a side surface thereof by being concavely formed, which is enclosed by a grip of a sensor holder according to the present invention. The grip groove 12 may be formed on at least one side surface of four side surfaces of the fastening unit 11. For example, the grip groove 12 may be formed along three side surfaces of the fastening unit except one side surface thereof, through which the cable 30 is withdrawn, but not limited thereto, the grip groove 12 may be provided on two opposite side surfaces of the fastener 11.

Here, it is preferred that the fastener 11 is provided at a center of the rear surface of the intraoral X-ray sensor 10, a flat area of the fastener 11 is more than 10% and less than 70% of an area of the rear surface of the intraoral X-ray sensor, and a height h1 of the fastener 11 is 5.0mm or more and a height h2 of the grip groove 12 is 3.0mm or more. Depending on this specific structure of the fastener 11, the intraoral X-ray sensor 10 shows sufficient binding force relative to a sensor holder according to the present invention.

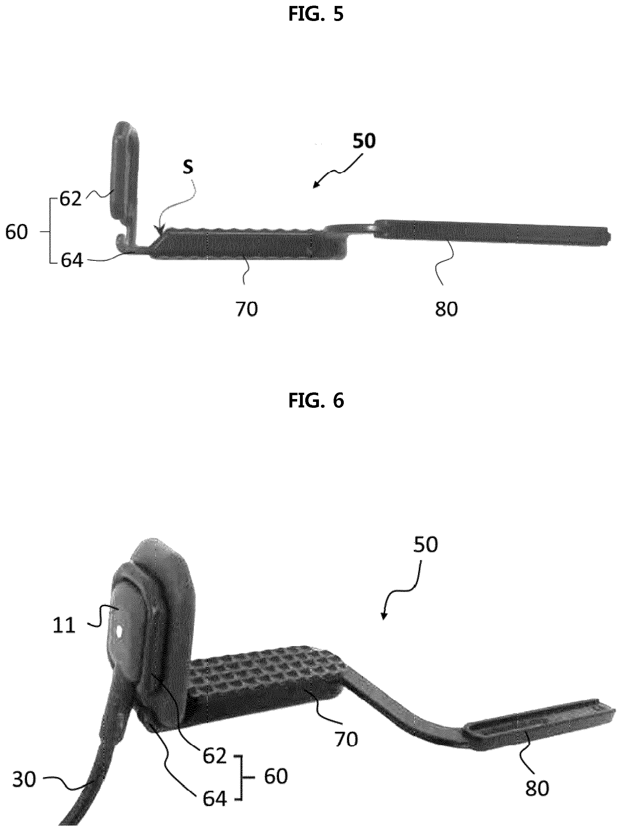

FIGS. 4 and 5 are views respectively showing a sensor holder 50 according to an embodiment of the present invention.

As shown in the drawings, the sensor holder 50 according to the present invention that is in a bar shape or a shape similar to a bar includes: a coupler 60 coupled with the intraoral X-ray sensor; a connector 70 connected to the coupler 60; and a handle 80 extending from the connector 70. Further, a predetermined location of the connector 70 may be integrally or detachably connected with a ring (see reference numeral 104 in FIGS. 1 and 2) that faces the intraoral X-ray sensor in parallel.

Here, particularly, the coupler 60 of the sensor holder 50 may include: a grip 62 configured to be fitted over a side surface of the fastener to enclose at least a part of the side surface; and a support 64 configured to connect the grip 62 and the connector 70 while covering at least parts of the rear surface and a side surface of the intraoral X-ray sensor. Further, the coupler 60 including the grip 62 and the support 64 may be made of a synthetic resin with a first hardness, or the rest of the elements, except the coupler 60, that is, the connector 70 and the handle 80, may be made of a synthetic resin with a second hardness greater than the first hardness. For example, the first hardness may be Shore hardness between D30 and D70, and the second hardness may be Shore hardness of D80 or more.

As described above, since the sensor holder 50 according to the present invention is configured such that the coupler 60 is made of a synthetic resin with hardness lower than the other elements, even though the subject's gums are strongly pressed by the intraoral X-ray sensor or the coupler 60 of the sensor holder 50 when intraoral radiography is performed, the coupler 60 appropriately relieves pressure with its elastic force, whereby it is possible to minimize pain to the subject.

Next, the connector 70 is configured to connect the coupler 60 and the handle 70, and a shape thereof may vary according to a target structure inside the subject's mouth. In other words, to facilitate intraoral radiography, the connector may be formed in various shapes.

Here, particularly, the connector 70 is provided with a bite configured to allow the subject to bite the same with the subject's mouth to hold a position when intraoral radiography is performed, and the coupler 60 may be particularly configured such that at least a part of the connector 70 connected to the support 64 is an inclined surface C with a height thereof gradually increased from the support 64 to the connector 70.

To be more specific, the connector 70 of the sensor holder 50 according to the present invention may be provided with the bite that allows the subject to bite with upper and lower anterior teeth when intraoral radiography is performed.

In this case, in order to allow the cable of the intraoral X-ray sensor to be safely withdrawn outside the mouth and to make the occlusal height of upper and lower teeth constant, at least a part of the connector 70, particularly, the bite may have a thickness thicker than the support 64. Accordingly, a step may occur at a junction between the support 64 and the connector 70, and in the process of decoupling the intraoral X-ray sensor from the coupler 60, the intraoral X-ray sensor may hit against the step, thereby causing damage.

Accordingly, the sensor holder 50 according to the present invention is configured such that the step occurring at the junction between the support 64 and the connector 70 is formed in an inclined surface with a height thereof gradually decreased from the connector 70 to the support 64, whereby it is possible to reduce shock that may be exerted onto the intraoral X-ray sensor when the intraoral X-ray sensor is coupled to or decoupled from the sensor holder, while facilitating disassembly and assembly thereof.

FIG. 6 is a view showing a combined state of the sensor holder 50 according to the present invention, and the intraoral X-ray sensor 10. See also FIGS. 3 to 5.

As shown in the accompanying drawings, the coupler 60 of the sensor holder 50 according to the present invention is fitted over the fastener 11 of the intraoral X-ray sensor 10.

Here, in order to engage the fastener 11 of the intraoral X-ray sensor 10 with the coupler 60 of the sensor holder 50, the grip 62 of the coupler 60 may be configured such that a part thereof is open to form an opening. Here, the opening may correspond to a side surface of the fastener 11, and it is preferred that a width of the opening is smaller than a length of the corresponding side surface of the fastener 11. To achieve this, opposite end portions of the grip 62 with the opening therebetween are bent in the same direction to face each other.

Accordingly, in the process of fitting the fastener 11 of the intraoral X-ray sensor 10 into the grip 62, the grip 62 allows the fastener 11 to be fitted thereinto by being opened in opposite directions based on the opening by an elastic force of the opening, and at the same time, is restored to an initial shape thereof by a restoring force to come into close contact with the grip groove 12 on. Further, the opposite end portions of the grip 62 that are bent to face each other with the opening therebetween serve as a stop protrusion that prevents separation of the fastener 11 while the fastener 11 is engaged with the grip.

Meanwhile, it is preferred that the opening of the grip 62 particularly faces a direction of the side surface with the cable 30 withdrawn therethrough, of the fastener 11 of the intraoral X-ray sensor 10, such that the cable 30 is withdrawn through the opening when the fastener 11 is coupled with the grip 62, whereby it is possible to prevent inconvenience caused by the cable 30.

As a result, even a part of the sensor holder 50 according to the present invention cannot exist on the X-ray path when being coupled with the intraoral X-ray sensor 10.

Accordingly, the sensor holder 50 according to the present invention is capable of preventing deterioration of the quality of an X-ray image caused when a part of the sensor holder is reflected in the X-ray image. Particularly, the sensor holder 50 according to the present invention is engaged with the fastener 11 provided on the rear surface of the intraoral X-ray sensor 10, whereby it is possible to minimize sizes of elements that are inserted into the subject's mouth when intraoral radiography is performed, and it is possible to further securely fasten the intraoral X-ray sensor 10.

Meanwhile, after intraoral radiography using the sensor holder 50 according to the present invention is performed, when the intraoral X-ray sensor 10 is decoupled from the sensor holder, the reverse process of coupling mentioned above is performed. Here, the user pushes or pulls the intraoral X-ray sensor 10 toward the connector 50 to separate the fastener 11 from the grip 62, wherein since the inclined surface is formed between the support 64 and the connector 70, it is possible to prevent damage caused when the intraoral X-ray sensor 10 hits against the connector 70.

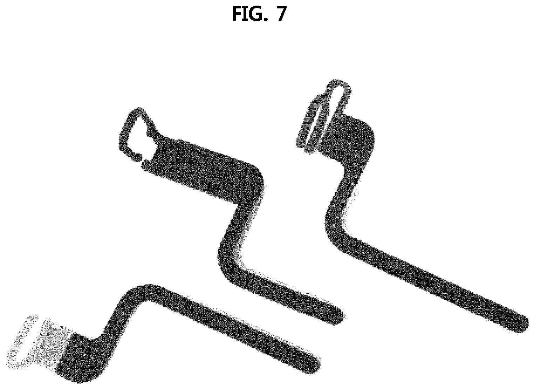

FIG. 7 is a view showing various shapes of the sensor holder according to the present invention.

As shown in the accompanying drawings, the connector of the sensor holder according to the present invention may be formed in various shapes according to a purpose of radiography, and if necessary, a shape of the support 64 may be changed. Further, different shapes of the sensor holders according to the present invention are formed as a set.

Although a preferred embodiment of the present invention has been described for illustrative purposes, those skilled in the art will appreciate that various modifications, additions and substitutions are possible, without departing from the scope and spirit of the invention as disclosed in the accompanying claims.

**REFERENCE SIGNS**

| | |
|---|---|
| 10: intraoral X-ray sensor | 11: fastener |
| 30: cable | 50: sensor holder |
| 60: coupler | 62: grip |
| 64: support | 70: connector |
| 80: handle | |

## Claims

1. A sensor holder (50) for an intraoral X-ray sensor (10) including a front surface receiving incident x-rays thereon, a rear surface opposite to the front surface, and a fastener (11) provided by protruding rearward on the rear surface, the sensor holder (50) comprising:
a coupler (60) including a grip (62) configured to be fitted over a side surface of the fastener (11) to enclose at least a part of the side surface and a support (64) configured to be connected to the grip (62) and face in a forward direction of the intraoral X-ray sensor (10) while covering at least parts of the rear surface and a side surface of the intraoral X-ray sensor (10);
a connector (70) configured to be connected to the support (64), and to extend in the forward direction of the intraoral X-ray sensor (10); and
a handle (80) configured to be connected to the connector (70),
wherein the coupler (60) is made of a synthetic resin material with a first hardness (L1), and at least one of the connector (70) and the handle (80) is made of a synthetic resin material with a second hardness (L2) greater than the first

2. The sensor holder (50) of claim 1, wherein the first hardness is Shore hardness between D30 and D70, and the second hardness is Shore hardness of D80 or more.

3. The sensor holder (50) of claim 1, wherein the connector (70) is provided with a bite configured to allow a subject to hold a position.

4. The sensor holder (50) of claim 1, wherein the connector (70) connected to the support (64) is configured such that at least a part thereof is inclined such that a height thereof is gradually increased from the support (64) to the connector (70).

5. The sensor holder (50) of claim 1, wherein the intraoral X-ray sensor (10) further includes a cable withdrawn from a side surface of the fastener (11), and
the grip (62) is configured such that a part thereof is open in a direction of the side surface of the fastener (11) for the cable.

6. The sensor holder (50) of claim 1, wherein the fastener (11) of the intraoral X-ray sensor (10) is provided with a grip (62) groove on at least a part of a side surface thereof, and
the grip (62) is configured to enclose the fastener (11) along the grip (62) groove.
